**Europäisches Patentamt**

**European Patent Office**

**Office européen des brevets**

(11) Veröffentlichungsnummer: **0 127 024**
A2

## (12) EUROPÄISCHE PATENTANMELDUNG

(21) Anmeldenummer: **84105192.3**

(22) Anmeldetag: **08.05.84**

(51) Int. Cl.³: **B 01 J 23/74**
B 01 J 23/84, B 01 J 23/76
B 01 J 37/02, C 07 C 1/04

(30) Priorität: **27.05.83 DE 3319254**

(43) Veröffentlichungstag der Anmeldung:
**05.12.84 Patentblatt 84/49**

(84) Benannte Vertragsstaaten:
**DE FR IT**

(71) Anmelder: **Bergwerksverband GmbH**
**Franz-Fischer-Weg 61**
**D-4300 Essen 13(DE)**

(72) Erfinder: **Hammer, Hans, Prof.Dr.-Ing.**
**Worringerweg 1**
**D-5100 Aachen(DE)**

(72) Erfinder: **Wübben, Peter, Dipl.-Ing.**
**Hans-Böckler-Allee 5**
**D-5100 Aachen(DE)**

(54) Verfahren zur Herstellung von Katalysatoren für die Erzeugung von ungesättigten Kohlenwasserstoffen aus Kohlenmonoxid-Wasserstoff-Gemischen.

(57) Die Erfindung betrifft einen Katalysator für die Erzeugung von ungesättigten Kohlenwasserstoffen aus Kohlenmonoxid-Wasserstoff-Gemischen sowie seine Verwendung zur Erzeugung von Ethylen und/oder Propylen-Gasgemischen, bei dem ein Katalysatorträger mit Acetylacetonaten der Übergangsmetalle, vorzugsweise des Eisens, Kobalts und Mangans sowie mit Promotoren imprägniert und bei erhöhter Temperatur im Wasserstoffstrom formiert worden ist, bei dem der Katalysatorträger aus Aktivkoksen mit hohem Anteil an Mikroporen unter 1 mm sowie Makroporen zwischen 800 − 5.000 mm besteht, eine Rütteldichte von 370 − 430 kg/m³ und eine BET-Oberfläche nach DIN 66 131 von 1.000 − 1.400 m²/g benutzt und nach der Imprägnierung im Wasserstoffstrom mit einer Raumgeschwindigkeit von 900 h⁻¹ bei einer Temperatur von 350°C behandelt wird. Die Verwendung der Katalysatoren erfolgt bei Kohlenmonoxid und Wasserstoff im Verhältnis 2:1 bis 1:1 enthaltenden Gasgemischen bei Temperaturen von 300 − 400°C und bei Drücken von 1 − 20 bar, die mit Raumgeschwindigkeiten zwischen 500 und 1.000 h⁻¹ über die Katalysatoren geleitet werden.

EP 0 127 024 A2

Die Erfindung betrifft ein Verfahren gemäß dem Oberbegriff des Anspruchs 1.

Es ist seit langem bekannt, daß aus Kohlenmonoxid-Wasserstoff-Gemischen an Katalysatoren mit den Übergangsmetallen Eisen und Kobalt durch heterogen-katalytische Umsetzung Kohlenwasserstoffe erzeugt werden können (DE-PSn 484 337 und 524 468). Es ist weiter bekannt, daß mit einer Zugabe von Mangan zu Eisenkatalysatoren eine Steigerung des Anteils an ungesättigten Kohlenwasserstoffen, vorzugsweise an Ethylen und Propylen, bei der Hydrierung von Kohlenmonoxid mittels Wasserstoff zu Kohlenwasserstoffen erzielt werden kann (DE-OS 25 07 647).

Ferner ist es allgemein bekannt, daß die Selektivität eines Katalysators neben chemischen Wirkungen auch von der Porenstruktur des Katalysators, beispielsweise von der Porenstruktur des für die katalytisch wirksamen Übergangsmetalle verwendeten Trägermaterials, abhängen kann. Es wurde erkannt, daß die Bildung kleiner Moleküle begünstigt und die Bildung großer Moleküle unterdrückt wird, wenn die innere Struktur des Katalysatormaterials den Transport kleiner Moleküle erlaubt, nicht aber den Transport großer Moleküle. Beispiele für solche Katalysatorträger sind insbesondere die Zeolithe. Es hat sich jedoch gezeigt, daß die saure Natur solcher Aluminiumsilikate bei der Erzeugung geradkettiger endständiger Olefine in nicht erwünschter Weise zu einer C-Strukturisomerisierung und zu einer Doppelbindungsisomerisierung führt (DE-PSn 484 337 und 524 468).

Der Erfindung liegt die Aufgabe zugrunde, die Hydrierung von Kohlenmonoxid so zu lenken, daß bevorzugt kleine Moleküle ungesättigter Kohlenwasserstoffe, wie beispielsweise Ethylen und Propylen gebildet werden, während gleichzeitig

...

die bei Aluminiumsilikaten auftretende Doppelbindungsisomerisierung und C-Strukturisomerisierung bei den im Produkt enthaltenen geradkettigen    Olefinen unterbunden
sind.

Diese Aufgabe wird erfindungsgemäß dadurch gelöst, daß bei
den eingangs genannten Katalysatoren der Katalysatorträger
aus Aktivkoksen mit einem hohen Anteil an Mikroporen mit
Porenradien unterhalb von 1 nm sowie an Makroporen mit Porenradien im Bereich von 800 - 5.000 nm besteht.

Die selektive Wirkung des Porensystems der Aktivkokse
kommt insbesondere dann voll zur Wirkung, wenn nach einem
weiteren Merkmal der Erfindung der Aktivkoks aus Formlingen mit einer Rütteldichte von 370 bis 430 kg/ m³ besteht,
die eine BET-Oberfläche nach DIN 66 131 von 1.000 bis
1.400 m²/g besitzen.

Ein weiteres Merkmal der Erfindung, um die Wirkung der Porenstruktur auf die Selektivität der Hydrierung mehr zur
Geltung zu bringen, besteht darin, daß die mit den Metall-
Acetylacetonaten imprägnierten Aktivkokse nach Abtrennung
der Imprägnierlösung mit Ethanol abgespült werden. Auf
diese Weise wird erreicht, daß nur das in das Innere des
Feinporensystems eingebrachte aktive Katalysatormaterial
zur selektiven Wirkung kommt.

Auch die Formierung des imprägnierten Aktivkokses (Katalysatorvorläufer) ist für die selektive Wirkung des erfindungsgemäßen Katalysators wichtig, indem die thermische
Zersetzung der Metall-Acetylacetonate im Wasserstoffstrom
mit einer Raumgeschwindigkeit von 900 h$^{-1}$ und bei einer
Temperatur von 350 °C durchgeführt wird.

...

Die Imprägnierung der Aktivkokse mit den Acetylacetonaten der aktiven Übergangsmetalle, insbesondere des Eisens und des Kobalts mit Zugabe von Mangan und unter Dotierung mit kleinen Mengen von Kupfer (als Acetylacetonat) und Kalium (als Karbonat) erfolgt unter Anwendung von vermindertem Druck, damit nach dem Evakuieren der Luft aus dem Porensystem die Imprägnierung der Poren mit der Ethanollösung der Katalysatormetalle bis in die Tiefe der Poren erfolgt und so geeignete Katalysatorvorläufer hergestellt werden können. Diese Katalysatorvorläufer werden durch die erfindungsgemäße Zersetzung der Metall-Acetylacetonate im Wasserstoffstrom zu aktiven Katalysatoren für die bevorzugt selektive Erzeugung von Ethylen und Propylen als Hydrierprodukte von Kohlenmonoxid neben nur geringen Mengen von höheren geradkettigen Olefinen als Nebenprodukten. Diese unerwartet hohe Selektivität der erfindungsgemäß hergestellten Katalysatoren liegt in der $C_2$-$C_3$-Fraktion überwiegend oberhalb von 95 %, häufig sogar nahe 100 %. Anhand der folgenden Ausführungsbeispiele sei die Herstellung der erfindungsgemäßen Katalysatoren näher beschrieben.

Die erfindungsgemäß hergestellten Katalysatoren werden beispielsweise für die Erzeugung von Ethylen und/oder Propylen-Gasgemischen verwendet, indem Kohlenmonoxid und Wasserstoff im Verhältnis 2 : 1 bis 1 : 1 enthaltende Gasgemische bei Temperaturen zwischen 300 und 400 °C, vorzugsweise bei 350°C, und bei Drücken von 1 bis 20 bar mit Raumgeschwindigkeiten zwischen 500 und 1.000 $h^{-1}$, vorzugsweise 600 $h^{-1}$, über die Katalysatoren geleitet werden.

...

**Beispiel 1**

Als Trägermaterial für den Katalysator dient ein Aktivkoks aus zylindrisch geformten Körnern mit einem mittleren Korndurchmesser von 3mm, der bei einer Rütteldichte von $400 \pm 20$ kg/ m³ und einem Gehalt an Wasser von 5 Gew.-% sowie an Asche von 6 Gew.-%, die eine BET-Oberfläche nach DIN 66131 von 1.200 m²/g besitzt. Dieser Aktivkoks zeigt weiter eine solche differentielle Porenradienhäufigkeitsverteilung, daß im Porenradien-Bereich unterhalb von 1,2 nm ein System von sehr engen Poren und im Bereich von 800 bis 5.000 nm ein kollektiv von Transportporen (Makroporen) vorliegt, welches zur raschen Versorgung des Inneren der Katalysatorkörner durch Diffusion dient. 40 g dieses Aktivkokses werden in einem Rundkolben mit Acetylacetonaten des Eisens und des Mangans im molaren Verhältnis 1 : 1 in einer solchen Menge versetzt, daß 10 %ige Lösungen beider Acetylacetonate vorliegen, wenn später 120 ml Ethanol zugefügt werden. Außerdem wird 0,1 Gew.-% Acetylacetonat des Kupfers, bezogen auf die aktiven Komponenten Eisen und Mangan, zugegeben. - Der Rundkolben wird dann mit aufgesetztem luftdicht verschlossenen Rückflußkühler mit dem Gemisch in einem auf 85 °C erwärmten Wasserbad auf einen Unterdruck von 5 mbar evakuiert und zwei Stunden auf diesem Unterdruck gehalten. Durch diese Behandlung wird der größte Teil der an den Feststoffen absorbierten Gase sowie das in den Poren des Aktivkokses befindliche Gas weitgehend entfernt. Nach zwei Stunden werden dann die erwähnten 120 ml Ethanol vorsichtig zugegeben, wobei das Ethanol Kalium- karbonat in einer Menge von 0,2 Gew.-% Kalium, bezogen auf die aktiven Komponenten Eisen und Mangan gleich 100, gelöst enthält.

...

Danach wird der Rundkolben durch den aufgesetzten Rückflußkühler belüftet und der Inhalt vier Stunden lang bei
einer Wasserbadtemperatur von 85 °C unter Rückfluß gekocht. Während dieser Zeit überzieht sich die innere und
äußere Oberfläche des Aktivkokses mit den aktiven Komponenten und Promotoren. Nach vier Stunden wird die Flüssigkeit abgenutscht und die Oberfläche der imprägnierten Aktivkokskörner mit Ethanol abgespült. Darauf läßt man den
Rest des Lösungsmittels verdampfen.

Anschließend erfolgt die Formierung des Katalysatorvorläufers durch eine thermische Behandlung im Wasserstoffstrom
mit einer Raumgeschwindigkeit von 900 h$^{-1}$ Die Temperatur
wird, während der Wasserstoff durch die Schüttung strömt,
von Zimmertemperatur auf 350°C hochgefahren und dann 12 h
konstant gehalten. Der fertige Katalysator enthält
2,9 Gew.-% Eisen und 2,6 Gew.-% Mangan.

Wird ein Gasgemisch aus gleichen Volumenteilen Kohlenmonoxid und Wasserstoff bei Normaldruck und 350 °C mit einer
Raumgeschwindigkeit von 600 h$^{-1}$ über den Katalysator geleitet, erhält man pro Nm³ umgesetztes Gasgemisch
34,69 g/Nm³ Ethylen, 0,38 g/Nm³ Propylen und 0,51 g/Nm³
Propan. Ethan wird nicht gebildet, dagegen 1,79 g/Nm³ Methan. Mithin beträgt die Selektivität zur Bildung von
Ethylen in der $C_2$-$C_3$-Fraktion 99,8 %.

Die Einzelumsätze betragen:
CO-Umsatz zu Kohlenwasserstoffen        23,8 %
CO-Umsatz zu $CO_2$                        0,4 %
Umsatz von CO und $H_2$                    27,0 %

...

Beispiel 2

Bei der Herstellung eines Katalysators unter den gleichen Bedingungen wie im Beispiel 1 beschrieben, jedoch mit dem Acetylacetonat des Kobalts anstelle von Mangan, wird ein fertiger Katalysator erhalten, der 2,9 Gew.-% Eisen und 4,3 Gew.-% Kobalt enthält.

Unter den gleichen Synthesebedingungen erhält man pro $Nm^3$-umgesetztes Gasgemisch 26,7 $g/Nm^3$ Ethylen, 0,38 $g/Nm^3$ Propylen und 0,32 $g/Nm^3$ Ethan. Propan wird nicht gebildet, dagegen 4,93 $g/Nm^3$ Methan. Die Selektivität zur Bildung von Ethylen in der $C_2$-$C_2$-Fraktion beträgt mithin 97,5 %.

Beispiel 3

Bei der Herstellung eines Katalysators unter den gleichen Bedingungen wie im Beispiel 1 beschrieben, jedoch mit den Acetylacetonaten des Kobalts und Mangans, wird ein fertiger Katalysator erhalten, der 4,7 Gew.-% Kobalt und 5,4 Gew.-% Mangan enthält.

Unter den gleichen Synthesebedingungen erhält man pro $Nm^3$ umgesetztes Gasgemisch 21,74 $g/Nm^3$ Ethylen, 0,28 $g/Nm^3$ Propylen und 0,26 $g/Nm^3$ Ethan. Propan wird nicht gebildet, dagegen 4,88 $g/Nm^3$ Methan. Die Selektivität zur Bildung von Ethylen in der $C_2$-$C_3$-Fraktion beträgt mithin 97,6 %.

Die Einzelumsätze betragen:

| | |
|---|---|
| CO-Umsatz zu Kohlenwasserstoffen | 24,1 % |
| CO-Umsatz zu $CO_2$ | 0,5 % |
| Umsatz von CO und $H_2$ | 29,5 % |

...

7     **0127024**

Beispiel 4

Über einen, wie im Beispiel 1 beschrieben, hergestellten Katalysator wird ein Gasgemisch aus gleichen Volumenteilen Kohlenmonoxid und Wasserstoff mit einer Raumgeschwindigkeit von 900 $h^{-1}$ bei sonst gleichen Synthesebedingungen geleitet. Man erhält pro $Nm^3$ umgesetztes Gasgemisch 26,7 $g/Nm^3$ Ethylen, 0,45 $g/Nm^3$ Propylen und 0,33 $g/Nm^3$ Propan. Ethan wird nicht gebildet, dagegen 1,37 $g/Nm^3$ Methan. Die Selektivität zur Bildung von Ethylen in der $C_2$-$C_3$-Fraktion beträgt mithin 97,1 %.

Die Einzelumsätze betragen:

| | |
|---|---|
| CO-Umsatz zu Kohlenwasserstoffen | 11,4 % |
| CO-Umsatz zu $CO_2$ | 0,28 % |
| Umsatz von CO und $H_2$ | 15,2 % |

BERGWERKSVERBAND GMBH

VERSUCHSBETRIEBE DER BERGBAU-FORSCHUNG GMBH

Essen, 20.05.1983

0127024

Verfahren zur Herstellung von Katalysatoren für die Erzeugung von ungesättigten Kohlenwasserstoffen aus Kohlenmonoxid-Wasserstoff-Gemischen

## Patentansprüche

1. Verfahren zur Herstellung von Katalysatoren für die Erzeugung von ungesättigten Kohlenwasserstoffen, vorzugsweise von Ethylen und/oder Propylen, aus Kohlenmonoxid-Wasserstoff-Gemischen durch Aufbringen von Acetylacetonaten der Übergangsmetalle, vorzugsweise des Eisens, Kobalts und Mangans oder deren binäre oder ternäre Mischungen, mit oder ohne weitere Promotoren in Form von Kupfer oder Kalium mittels eines Lösungsmittels, vorzugsweise Ethanol, auf einem Katalysatorträger und Formierung des Katalysators bei erhöhter Temperatur, <u>dadurch gekennzeichnet</u>, daß der Katalysatorträger aus Aktivkoksen mit einem hohen Anteil an Mikroporen mit Porenradien unterhalb von 1 nm sowie an Makroporen mit Porenradien im Bereich von 800 bis 5.000 nm besteht.

...

2. Verfahren nach Anspruch 1, <u>dadurch gekennzeichnet</u>, daß der Aktivkoks aus Formlingen mit einer Rütteldichte von 370 bis 430 kg/ m³ besteht, die eine BET-Oberfläche nach DIN 66 131 von 1.000 bis 1.400 m²/g benutzen.

3. Verfahren nach Ansprüchen 1 und 2, <u>dadurch gekennzeich- net</u>, daß die mit den Metall-Acetylacetonaten imprägnierten Aktivkoksformlinge nach Abtrennung der Imprägnierlösung mit Ethanol abgespült werden.

4. Verfahren nach Ansprüchen 1 bis 3, <u>dadurch gekennzeich- net</u>, daß die imprägnierten Aktivkoksformlinge (Katalysatorvorstufe) durch thermische Zersetzung der Metall-Acetylacetonate im Wasserstoffstrom mit einer Raumgeschwindigkeit von 900 h$^{-1}$ und bei einer Temperatur von 350 °C formiert werden.

5. Verwendung der nach Ansprüchen 1 bis 4 hergestellten Katalysatoren zur Erzeugung von Ethylen und/oder Propylen-Gasgemischen, <u>dadurch gekennzeichnet</u>, daß Kohlenmonoxid und Wasserstoff im Verhältnis 2 : 1 bis 1 : 1 enthaltende Gasgemische bei Temperaturen zwischen 300 und 400 °C, vorzugsweise bei 350 °C, und bei Drücken von 1 bis 20 bar mit Raumgeschwindigkeiten zwischen 500 und 1.000 h$^{-1}$, vorzugsweise 600 h$^{-1}$, über die Katalysatoren geleitet werden.

...